# EUROPEAN PATENT SPECIFICATION

(11) **EP 1 815 253 B1**
(45) Date of publication and mention of the grant of the patent: **08.07.2009**
(21) Application number: 05803833.2
(22) Date of filing: 16.11.2005
(51) Int. Cl.: G01N 33/569, G01N 33/50, G01N 33/58

(54) **ASSAY METHOD**
ASSAY-VERFAHREN
TECHNIQUE D'ESSAI

(30) Priority: 17.11.2004 GB 0425324
(43) Date of publication of application: 08.08.2007
(73) Proprietor: University Court of the University of Edinburgh, Edinburgh EH8 9YL (GB)
(72) Inventor: GREGORY, Christopher D., University of Edinburgh, Edinburgh EH16 4TJ (GB); DEVITT, Andrew, c/o Aston University, Birmingham B4 7ET (GB); TENNANT, Ian, Peterborough PE1 1NA (GB)
(74) Representative: Main, Malcolm Charles
(86) International application number: PCT/GB2005/004405
(87) International publication number: WO 2006/054068

(56) References cited:
- US-A- 5 637 465
- US-A- 5 834 196
- US-A1- 2003 203 365
- US-A1- 2004 022 731
- US-B1- 6 319 468
- VOGEL H J ET AL: "Towards a structure-function analysis of bovine lactoferricin and related tryptophan- and arginine-containing peptides" BIOCHEMISTRY AND CELL BIOLOGY 2002 CANADA, vol. 80, no. 1, 2002, pages 49-63, XP008059255 ISSN: 0829-8211

## Description

### Field of the Invention

The present invention relates to methods for the detection of dead and dying cells, reagents for use in such methods and uses of such reagents.

### Background to the Invention

Apoptosis is a fundamentally important process in normal development and homeostasis. Apoptosis is also known to play a role in many disease states. For example, apoptosis and defects in apoptotic pathways are believed to be particularly relevant to cancer, heart disease, stroke, Alzheimer's disease, ischaemia and autoimmune diseases. For example, cancers may result from a defect in apoptosis pathway, even in the absence of an increased proliferation rate.

Anti-cancer drug candidates failing to induce apoptosis are likely to have decreased clinical efficacy, making apoptosis assays important tools for high-throughput drug screening. The development of apoptosis modulating drugs requires robust assays for determining the presence of apoptotic and/or dead cells. Although a number of methods are available in the prior art to identify and/or quantify the presence of apoptotic and/or dead cells in a biological sample, for example a blood sample or a biopsy sample, the methods each suffer from specific disadvantages.

A number of methods are known in the art for assaying apoptosis. Nucleic acid stains may be used to identify apoptotic cells in cell populations, taking advantage of the characteristic breakdown of the nucleus during apoptosis, which results in collapse and fragmentation of the chromatin, degradation of the nuclear envelope and nuclear blebbing, resulting in the formation of "micronuclei".

Other methods used in the prior art include measurement of protease activity in cells, for example, measurement of the activity of effectors of the apoptosis pathway, such as caspase-3 and caspase-8. As a characteristic of apoptosis is the disruption of active mitochondria, mitochondrial stains may be used to identify apoptotic cells. Other apoptosis assays employ free radical probes or ion indicators to measure changes in apoptotic cells. One of the most commonly used assays of apoptosis is based on the use of the vascular anticoagulant annexin V. Annexin V is a phospholipid-binding protein that has a high affinity for phosphatidylserine. However, in viable cells, annexin V does not bind phosphatidylserine as it is located on the cytoplasmic surface of the cell membrane. In apoptotic cells, however, phosphatidylserine is externalised to the outer surface of the plasma membrane, where it can be bound by annexin V. However, the use of annexin V in assays for the detection of apoptosis suffers from a number of disadvantages. In particular, as binding of annexin V to phosphatidylserine is Ca²⁺ dependent, the choice of buffer used in assays is limited. This may result in a change of buffer prior to a sample being assayed, which can cause increased stress to cells and result in an increase in non-viable cells.

There is therefore a need for further assays which may be used for detecting the presence of non-viable cells in a sample, for example, for use in high throughput assays.

### Summary of the Invention

While testing a number of compounds as potential apoptotic cell indicators, the present inventors used a number of "control" substances. Surprisingly, on comparing the results using the "test" substances with the "control" substances, they discovered that one of the "control" substances, biotinylated milk unexpectedly selectively bound to dead and dying cells.

Accordingly, in a first aspect of the present invention, there is provided a method of detecting non-viable cells in a sample of cells, said method comprising the steps:
a) contacting a sample of said cells with a detectable reagent;
b) detecting binding of said reagent to cells of said sample;
wherein said reagent is milk, a milk product which selectively binds non-viable cells, or a milk component which selectively binds non-viable cells.

The non-viable cells may be dead or dying, for example apoptotic cells. In some embodiments of the invention, there will be no need to distinguish between different types of non-viable cells, for example, dead and apoptotic cells. However, in some embodiments, it may be useful to distinguish between such cells.

Thus, in one preferred embodiment of the invention, the method further comprises step (c) distinguishing cells that have undergone lysis from intact cells.

Any suitable method of distinguishing cells that have undergone lysis from intact cells known in the art may be used. For example, detectable markers of lysed cells may be used. In one embodiment, the detectable marker is propidium iodide. Other detectable markers which may be used include cell impermeant ethidium agents e.g ethidium homodimer-1; 7-aminoactinomycin D; TO-PRO-3 iodide (all available from Molecular Probes).

As described above, the inventors have found that milk or milk products, such as reconstituted dried milk may be used to selectively detect non-viable cells. Having established that such compositions can be used in this way, the inventors sought to identify particular components of milk which may be responsible for or contribute to the observed selective binding.

As demonstrated in the Examples, the inventors have found that a lipopolysaccharide-binding protein , lactoferrin, a major protein component of milk displays specific binding to dead or dying cells.

Accordingly, in a preferred embodiment of the invention, the detectable reagent for use in the invention is lipopolysaccharide-binding protein, for example lactoferrin, or an analogue thereof. In one embodiment, the reagent is lactoferrin.

Any lactoferrin may be used. It may be natural, for example purified from milk, or may be recombinantly produced. The lactoferrin may be from any mammalian species, for example, human, cow, sheep, goat. In one embodiment, the lactoferrin is human lactoferrin. In an alternative embodiment, the lactoferrin is bovine lactoferrin..

In the context of the present invention an analogue of lactoferrin is a protein having greater than 90% homology, preferably more than 95% homology , even more preferably greater than 97%, yet more preferably greater than 98% homology, most preferably greater than 99% homology with the amino acid sequence of human lactoferrin, while retaining lactoferrin activity. "Lactoferrin activity" is the ability to selectively bind non-viable cells, and preferably the ability to bind iron.

Homology comparisons may be conducted by eye, or more usually with the aid of readily available sequence comparison programs.

The percent identity of two amino acid sequences or of two nucleic acid sequences may be determined by aligning the sequences for optimal comparison purposes (e.g., gaps can be introduced in the first sequence for best alignment with the sequence) and comparing the amino acid residues or nucleotides at corresponding positions. The "best alignment" is an alignment of two sequences which results in the highest percent identity. The percent identity is determined by the number of identical amino acid residues or nucleotides in the sequences being compared (i.e., % identity = number of identical positions/total number of positions x 100).

The determination of percent identity between two sequences can be accomplished using a mathematical algorithm known to those of skill in the art. An example of a mathematical algorithm for comparing two sequences is the algorithm of Karlin and Altschul (1990) Proc. Natl. Acad. Sci. USA 87:2264-2268, modified as in Karlin and Altschul (1993) Proc. Natl. Acad. Sci. USA 90:5873-5877. The NBLAST and XBLAST programs of Altschul, et al. (1990) J. Mol. Biol. 215:403-410 have incorporated such an algorithm.

The invention is not limited to lactoferrin. The demonstration that the major milk component lactoferrin can be used to identify non-viable cells enables the identification of other milk components which share the property.

Accordingly, the detectable reagent for use in the invention may be any milk component which selectively binds non-viable cells. Preferably the milk component is a major component. In the context of the present invention, a milk component is considered to be a "major component" if, in whole milk, the component is present at a concentration of greater than 0.05 mg/ml, preferably greater than 0.1 mg/ml.

In preferred embodiments the detectable reagent is a lipopolysaccharide binding protein .

The detectable reagent for use in the invention may be detected using any suitable method in the art. For example, in one embodiment, the reagent may be detected using a reagent-specific antibody. In another embodiment, the reagent may be labelled. Any suitable label may be used. For example, labels which may be used include but are not limited to proteins such as ferritin, biotin, avidin and streptavidin and derivatives thereof, fluorescent markers, for example fluorescein, radioactive labels such as ¹²⁵I, ¹³¹I, ³²P, or ³⁵S, an enzymes such as alcohol dehydrogenase, peroxidase or alkaline phosphatase, dyes such as Evans Blue or Coomassie Brilliant Blue, detectable metals or detectable immunoglobulins.

As described above, the inventors have found that, in contrast to some of the apoptosis-detecting agents of the prior art, lactoferrin may be used to identify apoptotic cells in conditions of low concentrations of bivalent cations, for example, Ca²⁺-free conditions.

Thus in a preferred embodiment of the invention, the reagent is capable of descriminating between viable and non-viable cells in low Ca²⁺, preferably Ca²⁺-free conditions.

Accordingly, in one embodiment of the invention, the sample of said cells is contacted with the detectable reagent in the presence of less than 1 mM [Ca²⁺] . In one embodiment, the concentration of Ca²⁺ is less than 0.5mM, more preferably less than 100µM, for example less than 25µM. In one embodiment, the sample of said cells is contacted with the detectable reagent in the absence of free calcium. Furthermore, in further preferred embodiments, the concentration of other bivalent cations, such as Cd²⁺, Mg²⁺, Zn²⁺, or Co²⁺, is less than 0.5mM, more preferably less than 100µM, for example less than 25µM. In one embodiment, the sample of said cells is contacted with the detectable reagent in the absence of free bivalent cations. The method may comprise separating said apoptotic and/or dead cells from said sample.

In a second aspect, the invention provides a method of separating non-viable cells from viable cells in a biological sample, said method comprising:
a) contacting said sample with a detectable reagent;
b) isolating cells to which the reagent binds from said sample;
wherein said reagent is milk, a milk product which selectively binds non-viable cells, or a milk component which selectively binds non viable cells.

Furthermore, the invention enables the use of milk or milk components in the detection of non-viable cells in screens for the identification of compounds which modulate apoptosis in cells.

Accordingly, in a third aspect of the invention, there is provided a method of identifying a compound which modulates the apoptosis of cells, said method comprising
a) contacting a sample of cells with a candidate apoptosis-modulating compound,
b) contacting said sample of cells with a detectable reagent, wherein said reagent is milk, a milk product which selectively binds non-viable cells, or a milk component which selectively binds non-viable cells;
c) detecting binding of said reagent to cells of said sample, wherein binding is indicative of dead or apoptotic cells;
d) repeating steps (b) and (c) with a control sample of cells, which has not been exposed to the candidate apoptosis modulating compound;
wherein a difference in binding of the reagent between the cells which have been exposed to the candidate modulator and the control cells is indicative that the candidate modulator modulates apoptosis.

The invention may further be used in in vivo imaging of apoptotic cells. Accordingly, in a fourth aspect of the invention, there is provided a method of imaging cell death in a region of a subject in vivo, said method including the steps:
positioning the subject to whom had been administered a detectable reagent comprising milk, a milk product which selectively binds non viable-cells, or a milk component which selectively binds non-viable cells in the field of a scanning apparatus, wherein said milk, milk product or milk component is coupled to a label; detecting the location of the detectable reagent and label in the subject using the scanning apparatus.

Any label suitable for in vivo imaging may be used, with the scanning apparatus as appropriate to the label used. For example, the label may be a radionuclide with the scanning apparatus being a radiation emission detection apparatus. In another embodiment, the label may be a magnetic label, for example a magnetic resonance imaging (MRI) label, for example super paramagnetic nanoparticles, with an MRI scanner used as the scanning apparatus.

The pattern of radiation emission may be used to provide an indication of cell death in the region of the subject being scanned.

Any suitable radiation scanning apparatus may be used in this aspect of the invention. The choice of apparatus will be dependent on the choice of radiation emission. For example, the radiation scanning apparatus may be a positron emission tomography (PET) apparatus if the emitted radiation is positrons. If the emitted radiation comprises gamma ray radiation, the scanning apparatus may be a gamma ray radiation detector. Radionuclides useful with the method include Fluorine 18 if a positron emitter is required or Iodine 123, Iodine 131, Gallium 67, Indium 111, and Technetium 99, which are useful with standard gamma emission detection.

A fifth aspect of the invention comprises a kit for detecting apoptotic cells in a biological sample, said kit comprising
(i) a detectable reagent, wherein said reagent is milk, a milk product which selectively binds non-viable cells, or a milk component which selectively binds non-viable cells; and
(ii) a detectable marker for cells that have undergone lysis.

In a preferred embodiment of the fourth aspect of the invention, the kit further comprises a label for labelling the detectable reagent.

Preferred features of each aspect of the invention are as for each of the other aspects *mutatis mutandis*.

The invention will now be described further in the following non-limiting examples. Reference is made to the accompanying drawings in which:

### Brief description of the Drawings

Figure 1a shows cytometric analysis of binding of biotinylated milk component(s) to viable and non-viable human B cells.
Figure 1b shows cytometric analysis of binding of biotinylated milk component(s) to viable and non-viable murine hybridoma cells.
Figure 2 shows staining of cells with breast milk at a range of dilutions
Figure 3 shows staining of cells with breast milk at a range of dilutions.
Figure 4A shows dose dependent binding of bovine Lactoferrin (LFN) to non-viable murine hybridoma cells.
Figure 4B shows that binding of Lactoferrin is dose dependent with the control of BSA-biotin showing no dose-dependent binding. MFI=mean fluorescence intensity.
Figure 4C shows that binding of bovine Lactoferrin to non-viable murine hybridoma cells is inhibited by the addition of unlabelled lactoferrin.
Figure 4D shows that binding of bovine Lactoferrin to non-viable murine hybridoma cells is inhibited by the addition of unlabelled skimmed milk (SMP).
Figure 5 shows staining of a culture of murine hybridoma cells (containing dead cells). Staining is inhibited by the addition of unlabelled bovine Lactoferrin (figure 5a) or unlabelled bovine milk (reconstituted skimmed milk) (figure 5b).
Figure 6 shows the binding of Lf-bio to a culture of human B cells induced to die by apoptosis.
Figure 7 shows the binding of Lf-bio to a culture of human B cells induced to necrosis via heat-treatment.
Figure 8 shows staining of a culture of human B cells induced to apoptosis by irradiation with UV. The cells were stained with BM-bio in the presence or absence of a range of concentrations of one of the following unlabelled proteins: bovine Lf (Sigma); human Lf (Sigma); Conalbumin (substantially iron-free, Sigma) or conalbumin (iron-containing, Sigma).
Figure 9 shows staining of a culture of human B cells induced to apoptosis by irradiation with UV. The cells were stained with bovLf-bio in the presence or absence of a range of concentrations of one of the following unlabelled proteins: bovine Lf (Sigma); human Lf (Sigma); Conalbumin (substantially iron-free, Sigma) or conalbumin (not iron-free, Sigma).
Figure 10 shows staining of a culture of human B cells induced to apoptosis by irradiation with UV. Cells were stained with one of a range of concentrations of bovLf-FITC; humLf-FITC; BSA-FITC; bov-Lf-Alexa488 or BSA-Alexa488.
Figure 11 shows staining of a culture of human B cells induced to die by irradiation with UV. Cells were stained with biotinylated transferrin (Tf-bio; Sigma) at a range of concentrations.

### Examples

### Example 1 Bovine Milk can be used to detect dead and dying cells from different species

Reconstituted and biotinylated skimmed milk powder (Tesco) was incubated with a culture of human B cells that had been induced to die by apoptosis or a culture of murine hybridoma cells that contained spontaneous cell death. A saturated solution of skimmed milk powder was made in PBS, filtered through 0.2µm filter and biotinylated using a Sigma kit (Immunoprobe biotinylation kit) as per manufacturer's instructions. Biotinylated components of milk that had bound to cells were detected with streptavidin-Alexafluor 488 (Molecular Probes) and subsequent flow cytometric analysis as used by people skilled in the art.

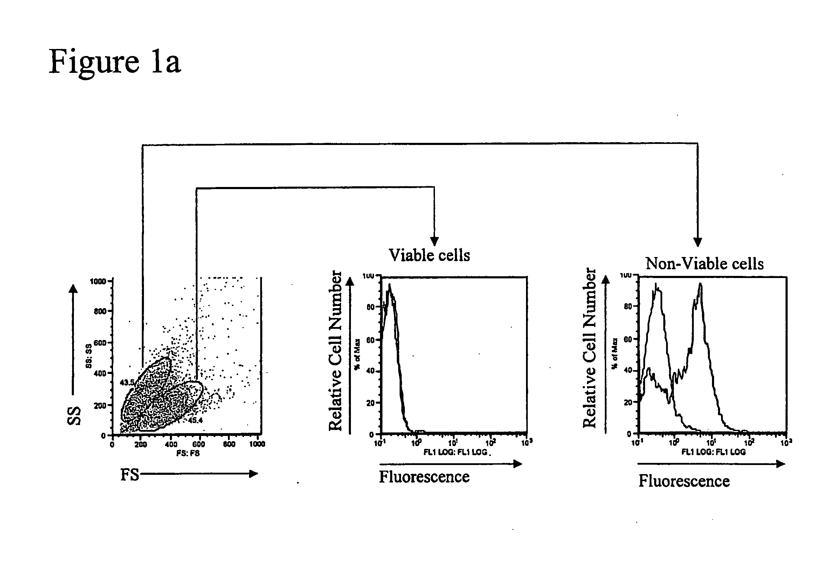

Figure 1a shows preferential binding of biotinylated milk component(s) to non-viable human B cells. Viable and non-viable cells were discriminated by their light scatter properties (FS v SS) as per methods used by people skilled in the art.

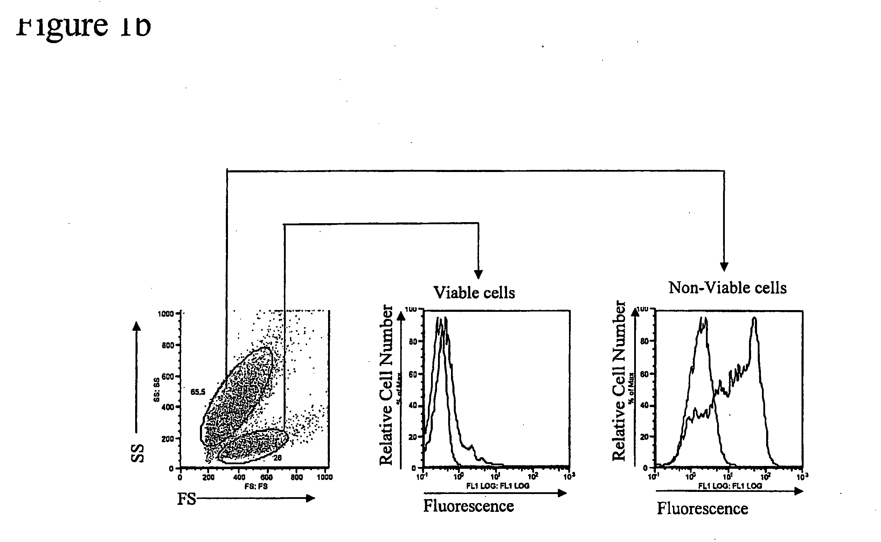

Figure 1b shows preferential binding of biotinylated milk component(s) to non-viable murine hybridoma cells. Viable and non-viable cells were discriminated by their light scatter properties (FS v SS) as per methods used by people skilled in the art.

### Example 2 Human Breast milk can be used to detect dead and dying cells

Human breast milk from a consenting volunteer donor was biotinylated as described: Complete human breast milk was centrifuged at high speed (14,000 x g) for 10 min. The upper lipid layer was discarded and the lower, cloudy infranatent harvested. The infranatent was filtered through a 0.2 µm filter prior to biotinylation with a biotinylation kit as per manufacturer's instructions (Immunoprobe Kit, Sigma). Biotinylated-breast milk was incubated with a culture of human B cells that had been induced to die by apoptosis. Biotinylated components of milk that had bound to cells were detected with streptavidin-phycoerythrin (Sigma Ltd) and subsequent flow cytometric analysis as used by people skilled in the art.

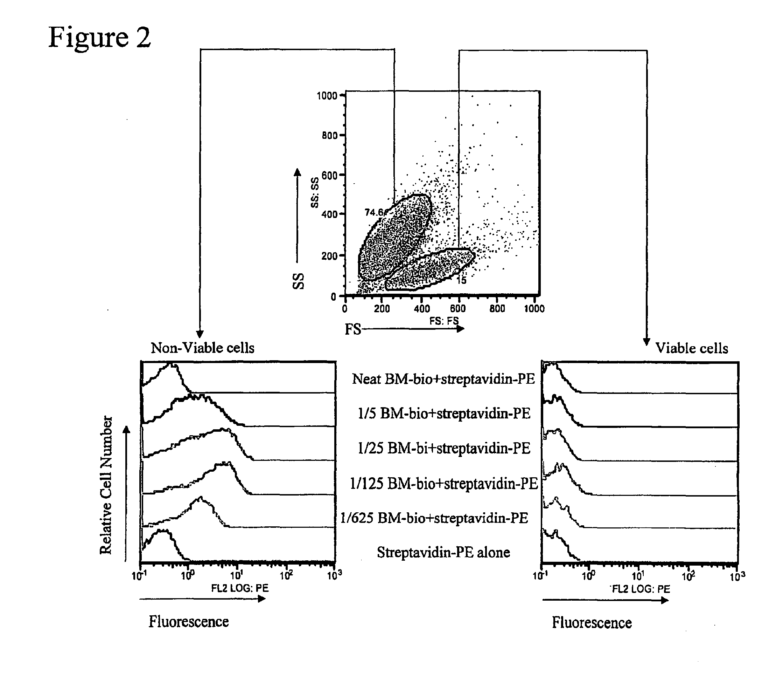

Figure 2 shows staining of cells with breast milk at a range of dilutions. The staining was specific for the non-viable cells as defined by their light scatter properties. Low staining of the cells was noted at high and low dilutions of the breast milk. This binding pattern is consistent with (a) an inhibitor being present in the milk that dilutes out more rapidly than the active binding factor; or (b) that the active ingredient can inhibit its own binding at high concentrations.

### Example 3 Inhibition of death with the pro-survival protein Bcl-2 inhibits binding of human breast milk

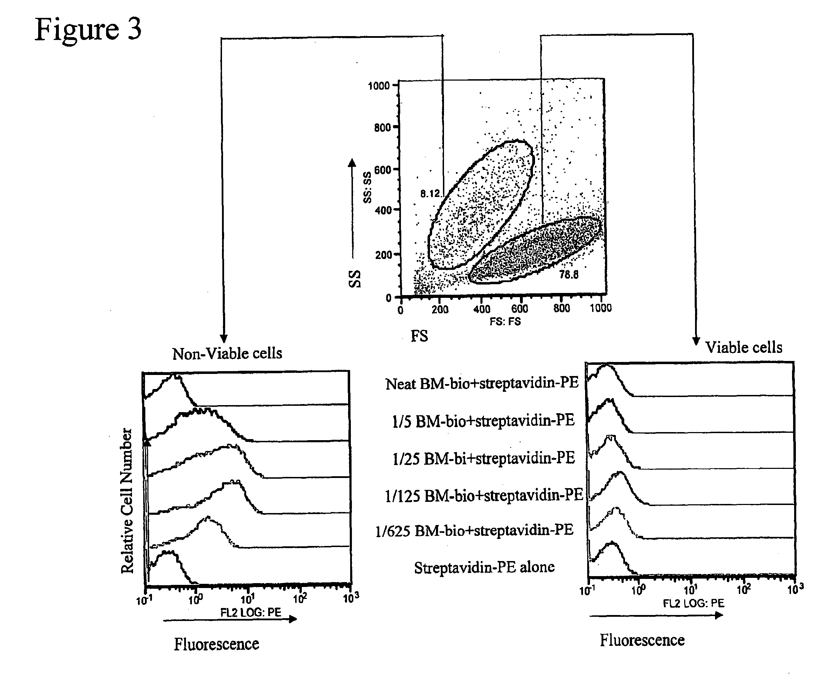

Human B cells (as used in figures 1& 2) were stably transfected with the cDNA for Bcl-2. Incubation of B/bcl-2 (B cells transfected with Bcl-2) with ionomycin (1µg/ml) resulted in limited death induction (unlike the non-transfected counterparts used in figures 1 & 2). Figure 3 shows staining of cells with breast milk at a range of dilutions. The staining was specific for the few non-viable cells as designated by their light scatter properties. Low staining of the cells was noted at high and low dilutions of the breast milk. Bcl-2 clearly inhibited ionomycin-induced death and staining with biotinylated breast milk. This result indicates that milk staining of cells was specific for non-viable cells.

### Example 4 Bovine lactoferrin binds dead hybridoma cells

If lactoferrin were the active ingredient in milk one would expect that purified Lf would bind dead cells. To this end bovine lactoferrin (Sigma) was biotinylated. Biotinylated bovine lactoferrin was incubated with a culture of murine hybridoma cells that contained spontaneous cell death. Biotinylated bovine lactoferrin that had bound to cells was detected with streptavidin-Alexafluor 488 (Molecular Probes) and subsequent flow cytometric analysis as used by people skilled in the art.

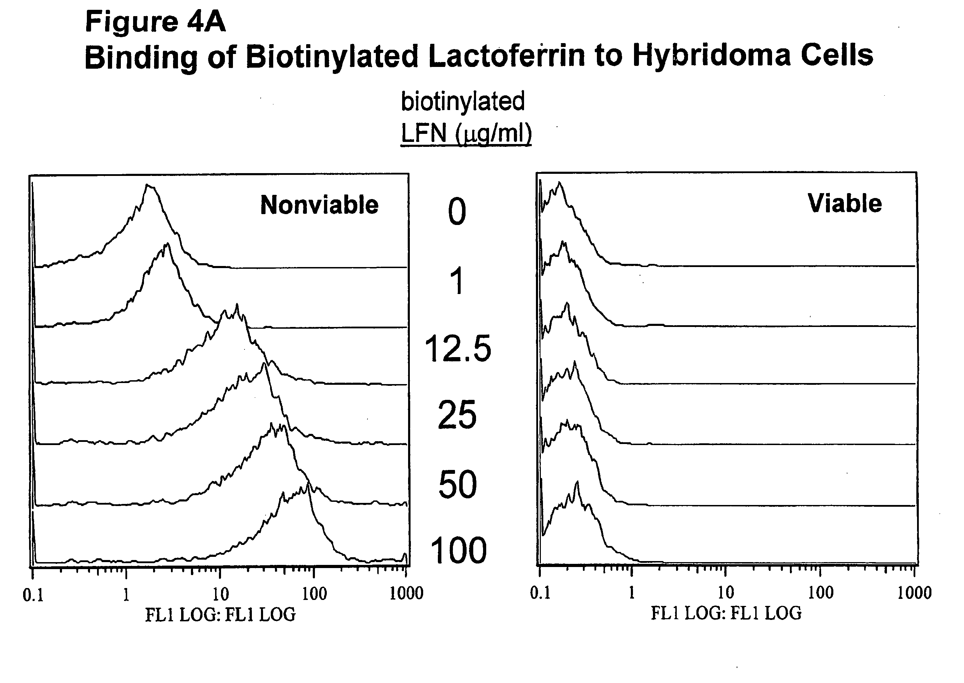

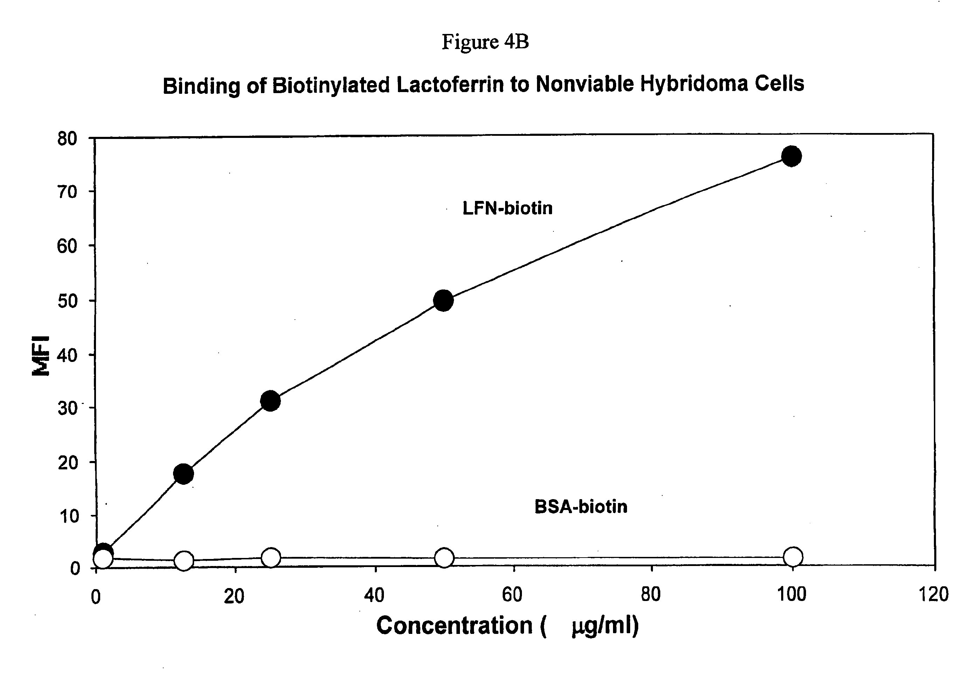

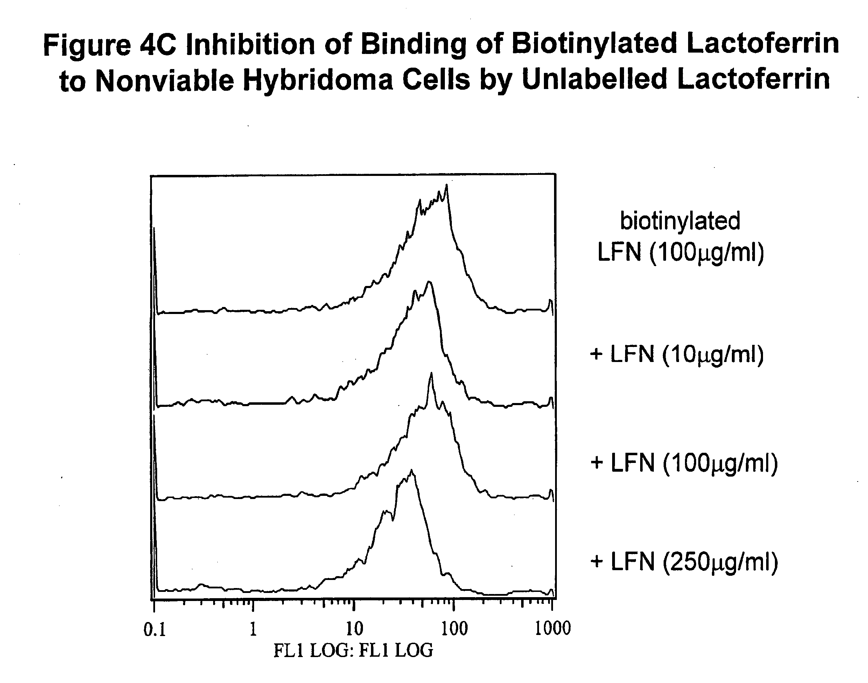

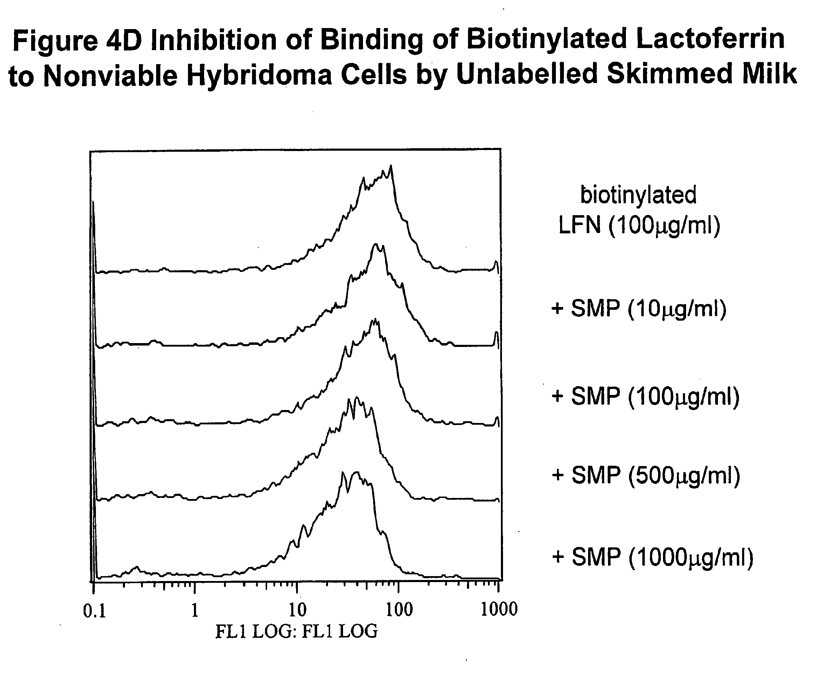

Figure 4A shows dose dependent binding of bovine Lactoferrin to non-viable murine hybridoma cells. Figure 4B shows that binding of Lactoferrin is dose dependent with the control of BSA-biotin showing no dose-dependent binding. Figure 4C shows that binding of bovine Lactoferrin to non-viable murine hybridoma cells is inhibited by the addition of unlabelled lactoferrin. Figure 4D shows that binding of bovine Lactoferrin to non-viable murine hybridoma cells is inhibited by the addition of unlabelled skimmed milk (SMP).

### Example 5 Binding of bovine Lactoferrin to dead hybridoma cells can be inhibited by non-labelled Lactoferrin or bovine milk.

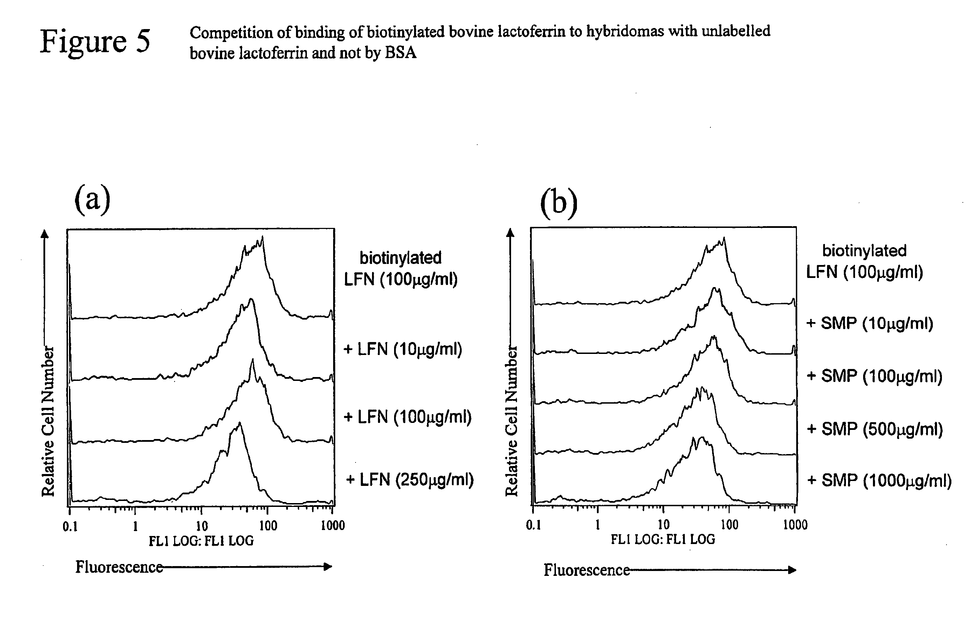

Figure 5 shows staining of a culture of murine hybridoma cells (containing dead cells). This staining is inhibited by the addition of unlabelled bovine Lactoferrin (figure 5a) or unlabelled bovine milk (reconstituted skimmed milk) (figure 5b). Inhibition occurs in a dose-dependent fashion.

### Example 6 Biotinylated bovine lactoferrin (Lf-bio) binds dead human B cells (both apoptotic and necrotic)

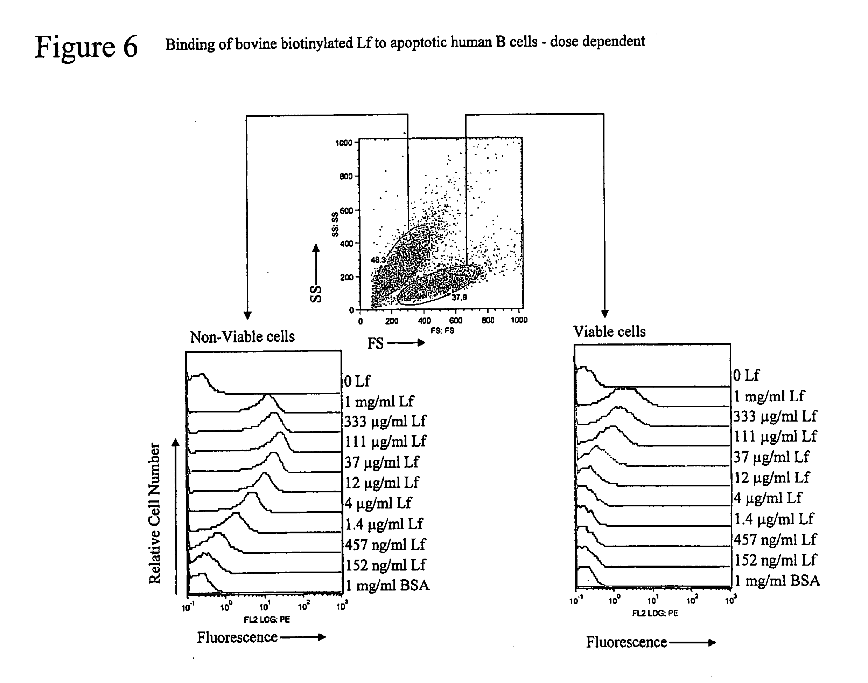

Figure 6 shows the binding of Lf-bio to a culture of human B cells induced to die by apoptosis by the addition of ionomycin (Calbiochem Ltd) to 1µg/ml. As noted with milk staining (figure 2), staining was low at high and low concentrations of Lf-bio. Further, the staining was preferentially confined to cells of the dead zone.

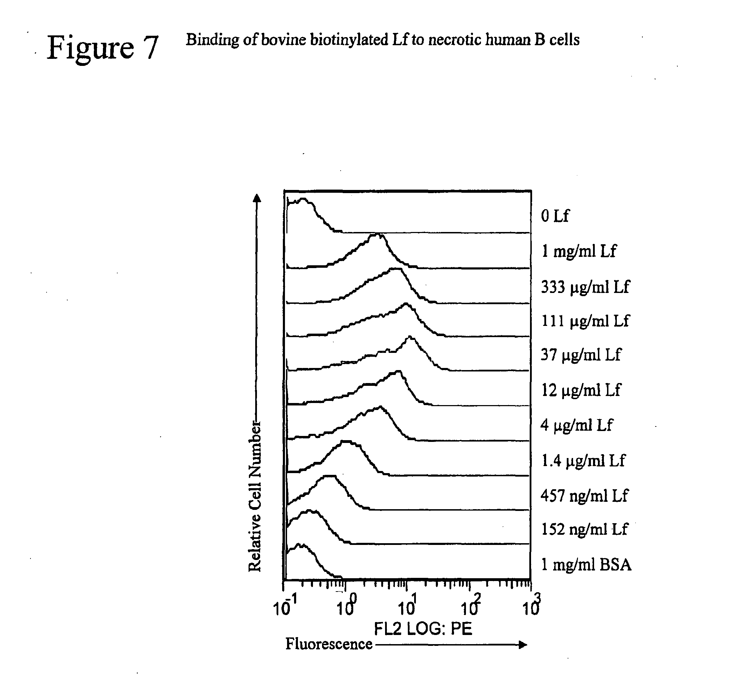

Figure 7 shows the binding of Lf-bio to a culture of human B cells induced to necrosis via heat-treatment. Cells were incubated at 56°C for 1 hour and the resultant cells were shown to be over 90% permeable to the dye propidium iodide. These necrotic cells stained with Lf-bio indicating that Lf-bio is capable of binding both apoptotic and necrotic cells.

### Example 7 Binding of biotinylated human breast milk (BM-bio) to dead cells can be inhibited by human lactoferrin or bovine lactoferrin but not by conalbumin or BSA

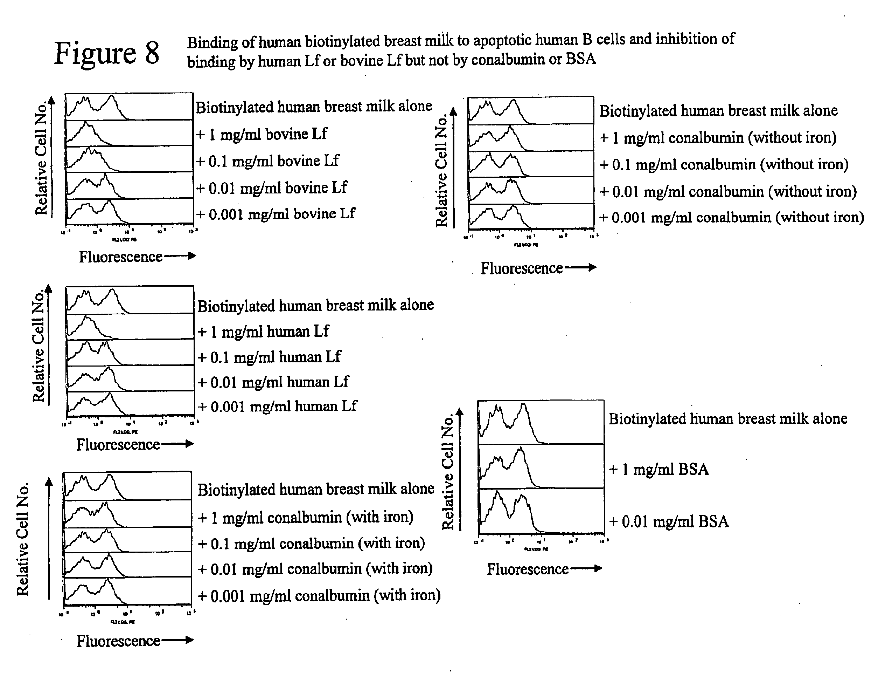

A culture of human B cells was induced to apoptosis by irradiation with UV (100mJ/cm²) followed by 24hours incubation at 37°C. Cells were stained with BM-bio in the presence or absence of a range of concentrations of one of the following unlabelled proteins: bovine Lf (Sigma); human Lf (Sigma); Conalbumin (substantially iron-free, Sigma) or conalbumin (iron-containing, Sigma). Figure 8 shows that Lf (bovine or human) inhibited the BM-bio staining of dead cells whilst the conalbumin preparations or the BSA showed no inhibitory effect. This result indicates that Lf from multiple species interacts with dead cells in substantially the same manner. This result further shows that dead cells induced by another mechanism (UV rather than ionomycin or heat) can be detected by BM-bio.

### Example 8 Binding of biotinylated bovine lactoferrin (bovLf-bio) to dead cells can be inhibited by human lactoferrin or bovine lactoferrin but not by conalbumin or BSA

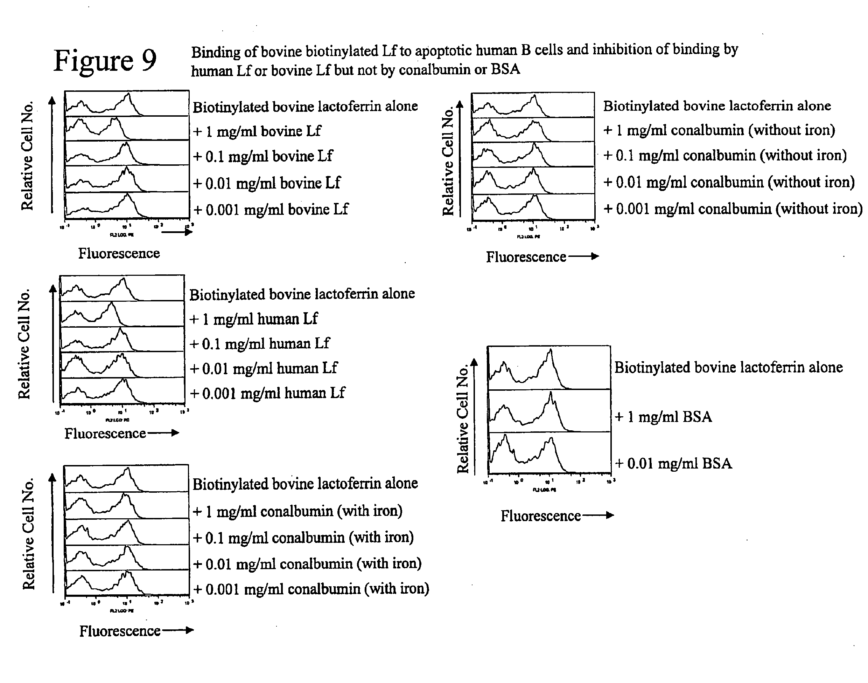

A culture of human B cells was induced to apoptosis by irradiation with UV (100mJ/cm²) followed by 24 hours incubation at 37°C. Cells were stained with bovLf-bio in the presence or absence of a range of concentrations of one of the following unlabelled proteins: bovine Lf (Sigma); human Lf (Sigma); Conalbumin (substantially iron-free, Sigma) or conalbumin (not iron-free, Sigma). Figure 9 shows that Lf (bovine or human) inhibited the bovLf-bio staining of dead cells whilst the conalbumin preparations or the BSA showed no inhibitory effect. This result indicates that Lf from multiple species interacts with dead cells in substantially the same manner. This result further shows that dead cells induced by another mechanism (UV rather than ionomycin or heat) can be detected by bovLf-bio.

### Example 9 Lactoferrin labelled in a variety of ways can be used to detect dead cells

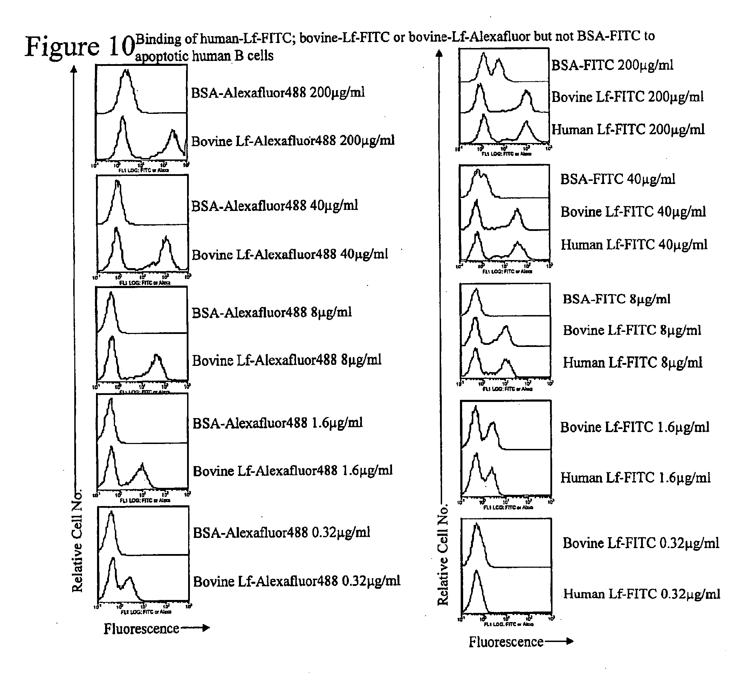

Results above indicate that Lf labelled with a biotin-tag and detected with streptavidin-fluorochrome conjugates bind to dead cells. In order to assess the ability of Lf to function for the detection of dead cells when tagged with different detection reagents purified bovine and human Lf were conjugated with FITC (fluorescein isothiocyanate). This was carried out using a FITC conjugation kit as per the manufacturer's instructions (Sigma). Purified bovine Lf was also separately conjugated with alexa-fluor 488 (Molecular Probes) as per the manufacturer's instructions. A culture of human B cells was induced to apoptosis by irradiation with UV (100mJ/cm²) followed by 24 hours incubation at 37°C. These cells were then stored on ice for 4-6 hours prior to staining. Cells were then stained with one of a range of concentrations of bovLf-FITC; humLf-FITC; BSA-FITC; bov-Lf-Alexa488 or BSA-Alexa488. Figure 10 shows strong staining of the induced population of cells with either bov-Lf-FITC or humLf-FITC. No such staining was noted with the control protein BSA-FITC. Similar results were obtained with alexa488-labelled bovLf. Strong staining of the induced population of cells was noted with bov-Lf-Alexa but not with BSA-Alexa. These results indicate that Lf may be labelled in a variety of ways without losing the capacity to bind dead cells.

### Example 10 Transferrin does not bind dead cells

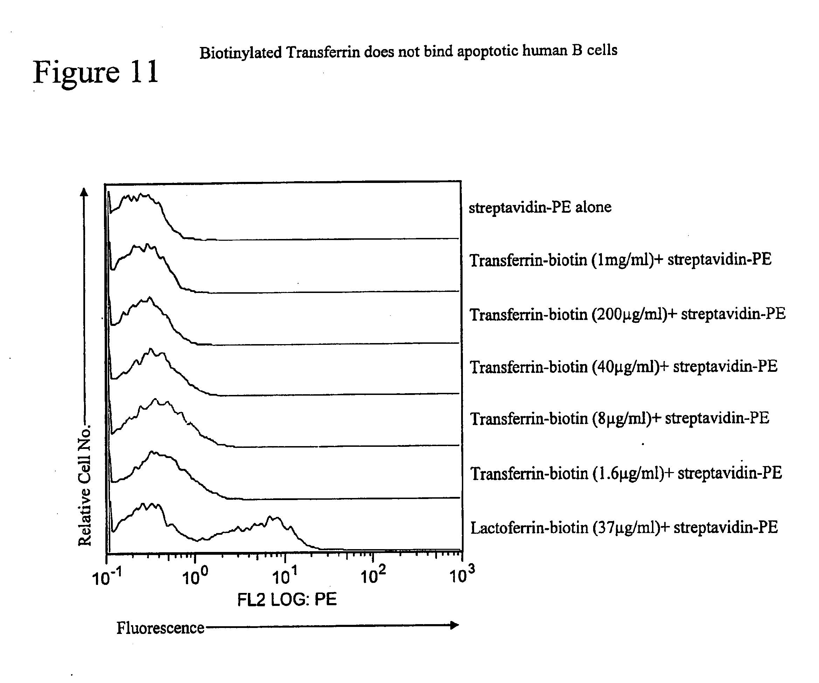

Lactoferrin, conalbumin and transferrin are all members of the transferrin family of proteins. They are reported to be iron-binding molecules. Figure 8 and 9 suggest that conalbumin does not bind dead cells in the same manner as Lf. Here biotinylated transferrin (Tf-bio; Sigma) was used at a variety of concentrations to stain a population of human B cells induced to die with UV irradiation and subsequent incubation at 37°C. Figure 11 shows that Tf-bio did not bind dead cells at a range concentrations from 1mg/ml to 1.6µg/ml whilst bovLf-bio successfully stained cells at 37µg/ml.

All documents referred to in this specification are herein incorporated by reference. Various modifications and variations to the described embodiments of the inventions will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the art are intended to be covered by the present invention.

## Claims

1. A method of detecting non-viable cells in a sample of cells, said method comprising the steps:
a) contacting a sample of said cells with a detectable reagent;
b) detecting binding of said reagent to cells of said sample;
wherein said reagent is milk, a milk product which selectively binds non-viable cells, or a milk component which selectively binds non-viable cells.

2. A method of separating non-viable cells from viable cells in a biological sample, said method comprising:
a) contacting said sample with a detectable reagent;
b) isolating cells to which the reagent binds from said sample;
wherein said reagent is milk, a milk product which selectively binds non-viable cells, or a milk component which selectively binds non-viable cells.

3. The method of claim 1 or claim 2, further comprising:
(c) distinguishing cells that have undergone lysis from intact cells.

4. The method according to claim 3, wherein cells that have undergone lysis are distinguished from intact cells by a detectable marker.

5. The method according to claim 4, wherein the detectable marker is propidium iodide.

6. The method according to any one of the preceding claims, further comprising separating said apoptotic or dead cells from said sample.

7. The method according to any one of the preceding claims, wherein the sample of said cells is contacted with the detectable reagent in the presence of less than 0.5mM Ca²⁺.

8. The method according to claim 7, wherein the sample of said cells is contacted with the detectable reagent in the absence of free Ca²⁺.

9. A method of identifying a compound which modulates the apoptosis of cells, said method comprising
a) contacting a sample of cells with a candidate apoptosis modulating compound,
b) contacting said sample of cells with a detectable reagent, wherein said reagent is milk, a milk product which selectively binds non-viable cells, or a milk component which selectively binds non-viable cells;
c) detecting binding of said reagent to cells of said sample, wherein binding is indicative of dead or apoptotic cells;
d) repeating steps (b) and (c) with a control sample of cells, which has not been exposed to the candidate apoptosis modulating compound;
wherein a difference in binding of the reagent between the cells which have been exposed to the candidate modulator and the control cells is indicative that the candidate modulator modulates apoptosis.

10. A method of imaging cell death in a region of a subject in vivo, said method including the steps:
positioning the subject, to whom had been administered a detectable reagent comprising milk, a milk product which selectively binds non-viable cells, or a milk component which selectively binds non-viable cells, in the field of a scanning apparatus, wherein said milk, milk product or milk component is coupled to a label;
detecting the location of the detectable reagent and label in the subject using the scanning apparatus.

11. The method according to any one of the preceding claims, wherein the detectable reagent is a major component of milk.

12. The method according to any one of the preceding claims, wherein the detectable reagent is a lipopolysaccharide binding protein (LBP).

13. The method according to claim 12, wherein the LBP is lactoferrin.

14. The method according to any one of claims 1 to 10 wherein the detectable reagent is reconstituted dried milk.

15. A kit for detecting apoptotic cells in a biological sample, said kit comprising
(i) a detectable reagent, wherein said reagent is milk, a milk product which selectively binds non-viable cells, or a milk component which selectively binds non-viable cells; and
(ii) a detectable marker for cells that have undergone lysis.

16. The kit according to claim 15, wherein the detectable marker is propidium iodide.

17. The kit according to claim 15 or claim 16, wherein the detectable reagent is a major component of milk.

18. The kit according to any one of claims 15, 16, or 17, wherein the detectable reagent is a lipopolysaccharide binding protein.

19. The kit according to claim 18, wherein the lipopolysaccharide binding protein is lactoferrin.

20. The kit according to claim 15 or claim 16 wherein the detectable reagent is reconstituted dried milk.

## Patentansprüche

1. Ein Verfahren zum Nachweisen nicht lebensfähiger Zellen in einer Zellprobe, wobei das Verfahren die folgenden Schritte beinhaltet:
a) In-Kontakt-Bringen einer Zellprobe mit einem nachweisbaren Reagens;
b) Nachweisen des Bindens des Reagens an Zellen der Probe;
wobei das Reagens Milch, ein Milchprodukt, das selektiv nicht lebensfähige Zellen bindet, oder ein Milchbestandteil ist, der selektiv nicht lebensfähige Zellen bindet.

2. Verfahren zum Trennen nicht lebensfähiger Zellen von lebensfähigen Zellen in einer biologischen Probe, wobei das Verfahren Folgendes beinhaltet:
a) In-Kontakt-Bringen der Probe mit einem nachweisbaren Reagens;
b) Isolieren von Zellen, an die sich das Reagens bindet, von der Probe;
wobei das Reagens Milch, ein Milchprodukt, das selektiv nicht lebensfähige Zellen bindet, oder ein Milchbestandteil ist, der selektiv nicht lebensfähige Zellen bindet.

3. Verfahren gemäß Anspruch 1 oder Anspruch 2, das ferner Folgendes beinhaltet:
(c) Unterscheiden von Zellen, die Lyse durchgemacht haben, von intakten Zellen.

4. Verfahren gemäß Anspruch 3, wobei Zellen, die Lyse durchgemacht haben, mittels eines nachweisbaren Markers von intakten Zellen unterschieden werden.

5. Verfahren gemäß Anspruch 4, wobei der nachweisbare Marker Propidiumiodid ist.

6. Verfahren gemäß einem der vorhergehenden Ansprüche, das ferner das Trennen der apoptotischen oder toten Zellen von der Probe beinhaltet.

7. Verfahren gemäß einem der vorhergehenden Ansprüche, wobei die Zellprobe in der Gegenwart von weniger als 0,5 mM Ca²⁺ mit dem nachweisbaren Reagens in Kontakt gebracht wird.

8. Verfahren gemäß Anspruch 7, wobei die Zellprobe in Abwesenheit von freiem Ca²⁺ mit dem nachweisbaren Reagens in Kontakt gebracht wird.

9. Verfahren zum Identifizieren einer Verbindung, welche die Zellapoptose moduliert, wobei das Verfahren Folgendes beinhaltet:
a) In-Kontakt-Bringen einer Zellprobe mit einer Apoptose modulierenden Kandidatenverbindung,
b) In-Kontakt-Bringen der Zellprobe mit einem nachweisbaren Reagens, wobei das Reagens Milch, ein Milchprodukt, das selektiv nicht lebensfähige Zellen bindet, oder ein Milchbestandteil ist, der selektiv nicht lebensfähige Zellen bindet;
c) Nachweisen des Bindens des Reagens an die Zellen der Probe, wobei das Binden auf tote oder apoptotische Zellen hinweist;
d) Wiederholen der Schritte (b) und (c) mit einer Kontrollzellprobe, welche nicht der Apoptose modulierenden Kandidatenverbindung ausgesetzt wurde;
wobei ein Unterschied beim Binden des Reagens zwischen den Zellen, die dem Kandidatenmodulator ausgesetzt wurden, und den Kontrollzellen darauf hinweist, dass der Kandidatenmodulator die Apoptose moduliert.

10. Ein Verfahren zum Abbilden von Zelltod in einem Bereich eines Objekts in vivo, wobei das Verfahren die folgenden Schritte umfasst:
Positionieren des Versuchsobjekts, dem ein nachweisbares Reagens verabreicht wurde, das Milch, ein Milchprodukt, das selektiv nicht lebensfähige Zellen bindet, oder einen Milchbestandteil, der selektiv nicht lebensfähige Zellen bindet, beinhaltet, im Bereich einer Abtastvorrichtung, wobei die Milch, das Milchprodukt oder der Milchbestandteil an eine Markierung gekoppelt ist;
Nachweisen der Stelle des nachweisbaren Reagens und der Markierung in dem Versuchsobjekt unter Verwendung der Abtastvorrichtung.

11. Verfahren gemäß einem der vorhergehenden Ansprüche, wobei das nachweisbare Reagens ein Hauptbestandteil von Milch ist.

12. Verfahren gemäß einem der vorhergehenden Ansprüche, wobei das nachweisbare Reagens ein Lipopolysaccharid bindendes Protein (LBP) ist.

13. Verfahren gemäß Anspruch 12, wobei das LBP Lactoferrin ist.

14. Verfahren gemäß einem der Ansprüche 1 bis 10, wobei das nachweisbare Reagens rekonstituierte Trockenmilch ist.

15. Ein Kit zum Nachweisen apoptotischer Zellen in einer biologischen Probe, wobei das Kit Folgendes beinhaltet:
(i) ein nachweisbares Reagens, wobei das Reagens Milch, ein Milchprodukt, das selektiv nicht lebensfähige Zellen bindet, oder ein Milchbestandteil ist, der selektiv nicht lebensfähige Zellen bindet; und
(ii) einen nachweisbaren Marker für Zellen, die Lyse durchgemacht haben.

16. Kit gemäß Anspruch 15, wobei der nachweisbare Marker Propidiumiodid ist.

17. Kit gemäß Anspruch 15 oder Anspruch 16, wobei das nachweisbare Reagens ein Hauptbestandteil von Milch ist.

18. Kit gemäß einem der Ansprüche 15, 16 oder 17, wobei das nachweisbare Reagens ein Lipopolysaccharid bindendes Protein ist.

19. Kit gemäß Anspruch 18, wobei das Lipopolysaccharid bindende Protein Lactoferrin ist.

20. Kit gemäß Anspruch 15 oder Anspruch 16, wobei das nachweisbare Reagens rekonstituierte Trockenmilch ist.

## Revendications

1. Une méthode pour déceler des cellules non viables dans un échantillon de cellules, ladite méthode comprenant les étapes de :
a) mettre un échantillon de dites cellules en contact avec un réactif décelable ;
b) déceler une liaison dudit réactif à des cellules dudit échantillon ;
dans laquelle ledit réactif est du lait, un produit laitier qui se lie de façon sélective à des cellules non viables, ou un composant du lait qui se lie de façon sélective à des cellules non viables.

2. Une méthode pour séparer des cellules non viables de cellules viables dans un échantillon biologique, ladite méthode comprenant :
a) mettre ledit échantillon en contact avec un réactif décelable ;
b) isoler des cellules auxquelles le réactif se lie dans ledit échantillon ;
dans laquelle ledit réactif est du lait, un produit laitier qui se lie de façon sélective à des cellules non viables, ou un composant du lait qui se lie de façon sélective à des cellules non viables.

3. La méthode de la revendication 1 ou de la revendication 2, comprenant en outre :
(c) distinguer des cellules qui ont subi une lyse de cellules intactes.

4. La méthode selon la revendication 3, dans laquelle des cellules qui ont subi une lyse sont distinguées de cellules intactes grâce à un marqueur décelable.

5. La méthode selon la revendication 4, dans laquelle le marqueur décelable est de l'iodure de propidium.

6. La méthode selon n'importe laquelle des revendications précédentes, comprenant en outre séparer lesdites cellules apoptotiques ou mortes dudit échantillon.

7. La méthode selon n'importe laquelle des revendications précédentes, dans laquelle l'échantillon de dites cellules est mis en contact avec le réactif décelable en la présence de moins de 0,5 mM de Ca²⁺.

8. La méthode selon la revendication 7, dans laquelle l'échantillon de dites cellules est mis en contact avec le réactif décelable en l'absence de Ca²⁺ libre.

9. Une méthode pour identifier un composé qui module l'apoptose de cellules, ladite méthode comprenant
a) mettre un échantillon de cellules en contact avec un composé de modulation d'apoptose candidat,
b) mettre ledit échantillon de cellules en contact avec un réactif décelable, ledit réactif étant du lait, un produit laitier qui se lie de façon sélective à des cellules non viables, ou un composant du lait qui se lie de façon sélective à des cellules non viables ;
c) déceler une liaison dudit réactif à des cellules dudit échantillon, la liaison étant indicatrice de cellules mortes ou apoptotiques ;
d) répéter les étapes (b) et (c) avec un échantillon témoin de cellules, lequel n'a pas été exposé au composé de modulation d'apoptose candidat ;
dans laquelle une différence de liaison du réactif entre les cellules qui ont été exposées au modulateur candidat et les cellules témoins est indicatrice du fait que le modulateur candidat module l'apoptose.

10. Une méthode pour imager la mort cellulaire dans une région d'un sujet in vivo, ladite méthode incluant les étapes de :
positionner le sujet, auquel il aura été administré un réactif décelable comprenant du lait, un produit laitier qui se lie de façon sélective à des cellules non viables, ou un composant du lait qui se lie de façon sélective à des cellules non viables, dans le champ d'un appareil de balayage, ledit lait, produit laitier ou composant du lait étant couplé à un traceur ;
déceler l'emplacement du réactif décelable et du traceur chez le sujet en utilisant l'appareil de balayage.

11. La méthode selon n'importe laquelle des revendications précédentes, dans laquelle le réactif décelable est un composant majeur du lait.

12. La méthode selon n'importe laquelle des revendications précédentes, dans laquelle le réactif décelable est une protéine de liaison aux lipopolysaccharides (LBP).

13. La méthode selon la revendication 12, dans laquelle la LBP est de la lactoferrine.

14. La méthode selon n'importe laquelle des revendications 1 à 10 dans laquelle le réactif décelable est du lait en poudre reconstitué.

15. Un kit destiné à déceler des cellules apoptotiques dans un échantillon biologique, ledit kit comprenant
(i) un réactif décelable, ledit réactif étant du lait, un produit laitier qui se lie de façon sélective à des cellules non viables, ou un composant du lait qui se lie de façon sélective à des cellules non viables ; et
(ii) un marqueur décelable pour des cellules qui ont subi une lyse.

16. Le kit selon la revendication 15, dans lequel le marqueur décelable est de l'iodure de propidium.

17. Le kit selon la revendication 15 ou la revendication 16, dans lequel le réactif décelable est un composant majeur du lait.

18. Le kit selon n'importe laquelle des revendications 15, 16 ou 17, dans lequel le réactif décelable est une protéine de liaison aux lipopolysaccharides.

19. Le kit selon la revendication 18, dans lequel la protéine de liaison aux lipopolysaccharides est de la lactoferrine.

20. Le kit selon la revendication 15 ou la revendication 16 dans lequel le réactif décelable est du lait en poudre reconstitué.
